# EUROPÄISCHE PATENTSCHRIFT

(11) **EP 0 695 553 B1**
(45) Veröffentlichungstag und Bekanntmachung des Hinweises auf die Patenterteilung: **24.03.1999**
(21) Anmeldenummer: 94112154.3
(22) Anmeldetag: 03.08.1994
(51) Int. Cl.: A61L 9/03, A01M 1/20

(54) **Elektrische Vorrichtung zum Verdunsten von Wirkstoffen**
Electric vaporizer for active substances
Appareil électrique de vaporisation de substances actives

(43) Veröffentlichungstag der Anmeldung: 07.02.1996
(73) Patentinhaber: Steinel GmbH & Co. KG, D-33442 Herzebrock-Clarholz (DE)
(72) Erfinder: Steinel jun., Heinrich Wolfang, D-86825 Bad Wörishofen (DE)
(74) Vertreter: Grünecker, Kinkeldey, Stockmair & Schwanhäusser Anwaltssozietät

(56) Entgegenhaltungen:
- EP-A- 0 290 159
- WO-A-88/05310
- DE-U- 9 014 309
- DE-U- 9 104 709

## Beschreibung

Die vorliegende Erfindung bezieht sich auf eine elektrische Vorrichtung zum Verdunsten von Wirkstoffen gemäß dem Oberbegriff des Patentanspruchs 1.

Aus der EP-A-0 290 159 ist eine Vorrichtung zum Verdunsten von Wirkstoffen gemäß dem Oberbegriff des Patentanspruchs 1 bekannt. Bei dieser Vorrichtung befindet sich ein zu beheizender Körper in Anlage mit einer ersten Elektrode für zwei elektrische Heizelemente. Eine zweite Elektrode ist halbringförmig ausgebildet und umklammert von außen die beiden Heizelemente, wodurch diese gegen die innenliegende erste Elektrode und den zu beheizenden Körper gedrückt werden.

Der vorliegenden Erfindung liegt die Aufgabe zugrunde, eine solche Vorrichtung derart weiterzubilden, daß sie einfach herzustellen ist und mit geringer elektrischer Verlustleistung arbeitet.

Diese Aufgabe wird von einer Vorrichtung mit den Merkmalen des Anspruchs 1 gelöst. Die erfindungsgemäße Lösung zeichnet sich dadurch aus, daß das Heizelement ein PTC-Element ist, welches zwischen zwei Elektroden eingeklemmt ist und daß wenigstens eine der beiden Elektroden nahe dem Wärmetauscher angeordnet und einstückig mit diesem ausgebildet ist.

Durch diese Ausbildung der Heizung wird erreicht, daß die Elektroden für das elektrische Heizelement gleichzeitig dazu dienen, die von dem Heizelement freigesetzte Wärme dem Wärmetauscher zuzuführen. Durch die einstückige Ausbildung der Elektroden mit dem Wärmetauscher besteht die Vorrichtung aus weniger Teilen, wodurch sie einfach und billig herzustellen ist. Weiterhin besitzt die erfindungsgemäße Vorrichtung eine gute Wärmeleitfähigkeit zwischen dem Heizelement und dem Wärmetauscher, und daher die Verlustleistung gering gehalten werden kann.

In einer einfachen Ausgestaltung weist die Vorrichtung einen mit dem Gehäuse verbindbaren Behälter für die zu verdunstende Substanz auf, in dem ein Docht gelagert ist, und der Wärmetauscher besitzt einen Durchgangskanal, der den Docht umschließt.

Vorzugsweise ist der Wärmetauscher ringförmig ausgebildet mit einem tiefgezogenen Abschnitt, der sich koaxial zum Docht erstreckt. Bei dieser Ausführungsform des Wärmetauschers erfolgt eine gute Auskopplung der Wärme zum Docht, wodurch ein guter Wirkungsgrad der Vorrichtung erzielt wird.

In einer dazu alternativen Ausführungsform ist der Wärmetauscher als eine ebene Heizfläche ausgebildet, die als Auflagefläche für einen Behälter für die zu verdunstende Substanz dient. Bei dieser Ausführungsform ist durch die unmittelbare Nähe des Behälters an der Heizfläche eine direkte Einwirkung der vom Wärmetauscher freigesetzten Wärme auf die gesamte zu verdunstende Substanz möglich.

Vorzugsweise sind die Elektroden und/oder der Wärmetauscher mit einstückig daran ausgebildeten Verbindungsleitungen versehen. Durch die einstückige Ausbildung von Elektroden, Wärmetauscher und Verbindungsleitungen vermindert sich die Anzahl der Teile, was die Herstellung der Vorrichtung vereinfacht, da die üblichen Litzenverbindungen entfallen und nahezu alle elektrischen Teile auf einem gemeinsamen Stanzteil hergestellt werden können.

Es ist von Vorteil, wenn die Verbindungsleitungen, der Wärmetauscher und die Elektroden einstückig auf einem Stanzblech ausgebildet sind, das nach dem Stanzen erforderlichenfalls abgebogen und an den Stellen, an denen eine erhöhte Formsteifigkeit bzw. eine Wärmeisolierung erforderlich ist, mit einem Kunststoff umspritzt ist. Dies vereinfacht die Herstellung der Vorrichtung erheblich, da sich alle Teile aus einem ebenen Werkstück unter Einsatz von Produktionsmaschinen herstellen lassen.

In einer weiteren bevorzugten Ausgestaltung ist zwischen den Verbindungsleitungen und den Elektroden oder dem Wärmetauscher ein Bereich mit vermindertem Leitungsquerschnitt ausgebildet, der einen ausreichenden elektrischen Kontakt für die Elektroden herstellt, die Wärme jedoch nur sehr schlecht leitet. Dadurch wird ein Wärmeverlust über die Verbindungsleitungen weitgehend vermieden.

In einer weiteren Ausführungsform der Erfindung sind die Elektroden auf den voneinander abgewandten Seiten von dem Kunststoff umgeben und weisen auf den einander zugewandten Seiten an ihrem äußeren Rand einen Steg auf. Durch diese Ausgestaltung ist das zwischen den Elektroden angeordnete PTC-Element formschlüssig zwischen den Stegen gehalten, wodurch es sich erübrigt, zusätzliche Halteelemente für das Heizelement vorzusehen.

Vorzugsweise ist zwischen einer Elektrode und dem PTC-Element ein elastisches Element angeordnet und ein Klemmelement umgreift von außen die zwei Elektroden und das zwischen ihnen angeordnete PTC-Element. Bei dieser Anordnung wird ein inniger Kontakt der Elektroden zu dem PTC-Element erreicht, was nicht nur zu einer sicheren elektrischen Verbindung führt, sondern auch die Wärmeauskopplung zwischen dem Heizelement und den Elektroden verbessert.

Die vorliegende Erfindung soll nun anhand bevorzugter Ausführungsbeispiele unter Bezugnahme auf die Zeichnungen erläutert werden, die zeigen:
- Fig. 1: eine Seitenansicht der Vorrichtung,
- Fig. 2: eine Seitenansicht der in dem Gehäuse gelagerten Heizung der Vorrichtung,
- Fig. 3: eine Sicht von oben auf die Heizung, die in Fig. 2 dargestellt ist,
- Fig. 4: eine Seitenansicht auf den Anschlußstecker und das erste Drehkupplungsteil der vorliegenden Erfindung,
- Fig. 5: eine Ansicht von vorne auf das erste Drehkupplungsteil,
- Fig. 6: eine Darstellung eines Stanzblechteils, das wesentliche Teile der Vorrichtung enthält, und
- Fig. 7: eine vergrößerte Schnittdarstellung einer Elektrode mit daran integriertem Wärmetauscher.

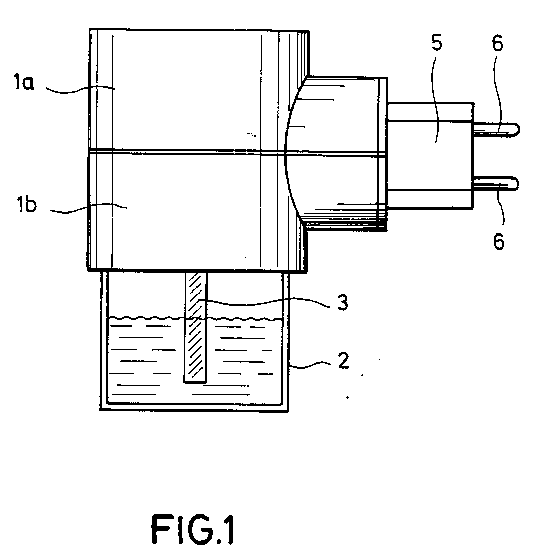

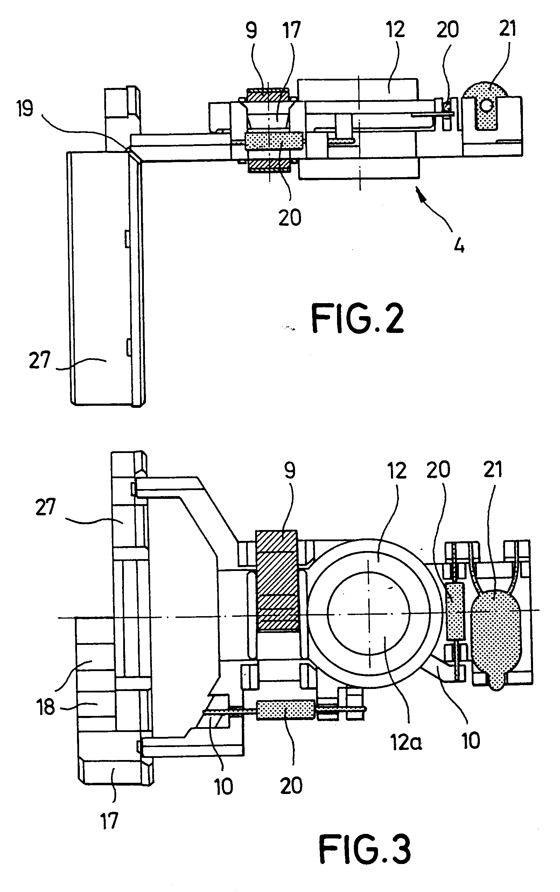

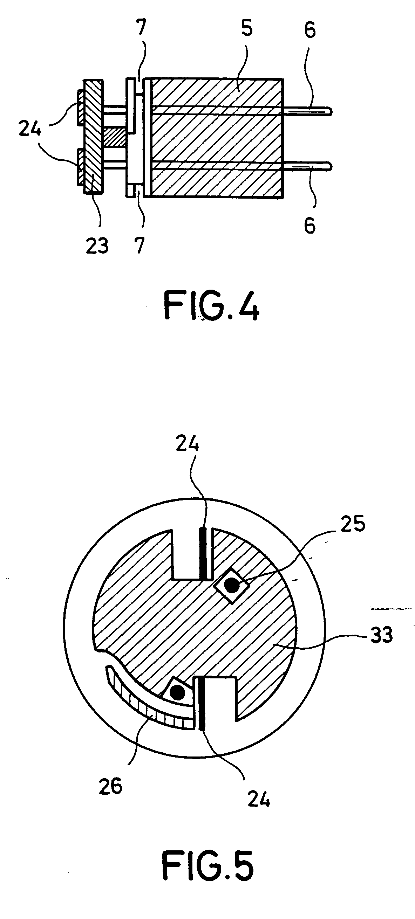

Gemäß Fig. 1 weist die Vorrichtung ein aus zwei Gehäusehalbschalen 1a, 1b bestehendes Gehäuse auf, mit einem daran befestigbaren Behälter 2, in der sich die zu verdunstende Substanz, beispielsweise eine Flüssigkeit mit darin gelösten wirkstoffen befindet. Die Befestigungsmittel zwischen dem Gehäuse und dem Behälter 2 bestehen aus einem am oberen Ende des Behälters angeordneten Außengewinde und einem entsprechenden in der unteren Gehäusehalbschale 1b ausgebildeten Innengewinde. In dem Behälter 2 ist ein Docht 3 gelagert, der aus Kohlefasern oder Textilfäden besteht. Über den Docht wird die zu verdunstende Flüssigkeit zu der in den Fig. 2 und 3 näher dargestellten Heizung 4 transportiert. In dem Gehäuse ist ein Anschlußstecker 5 mit zwei daran befestigten Steckkontakten 6 drehbar gelagert. Wie aus Fig. 4 ersichtlich, weist der Anschlußstecker eine umlaufende Ringnut 7 auf, die bei geschlossenem Gehäuse in eine entsprechend ausgeformte Randkante der Gehäuseschalen 1a, 1b eingreift.

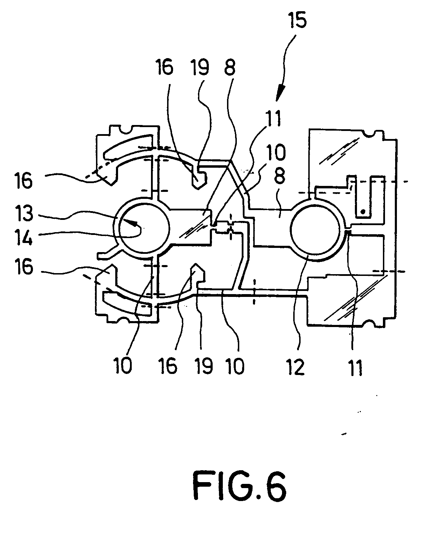

Die Heizung 4 umfaßt ein zwischen zwei übereinander angeordneten Elektroden 8 eingeklemmtes PTC-Heizelement, welches in den Fig. 2 und 3 nicht direkt sichtbar ist, da die Elektroden 8 größtenteils von einem wärmebeständigen und isolierenden Kunststoffmaterial umspritzt sind und eine freie Sicht auf das PTC-Element verhindern. Die Elektroden sind auf den voneinander abgewandten Seiten in dem Kunststoffmaterial eingebettet. Die einander zugewandten Seiten weisen eine Kontaktfläche auf, die groß genug ist, um das PTC-Element vollständig zu kontaktieren. Entlang des äußeren Randes der Elektroden ist ein umlaufender Steg ausgebildet, wobei die Stege auf der oberen und unteren Elektrode so angeordnet sind, daß sie ineinander eingreifen. Dadurch bildet sich ein abgeschlossener Hohlraum, in dem das PTC-Element formschlüssig gehalten ist. Zwischen der unteren Elektrode und dem PTC-Element befindet sich ein elastisches, elektrisch leitendes Element, welches zusammen mit einer von außen die Elektroden umfassenden U-förmigen Klemmfeder 9 für einen guten Kontakt zwischen den Elektroden und dem PTC-Element sorgt. Der elektrische Teil der Heizung umfaßt weiterhin Verbindungsleitungen 10, von denen ein Teil über einen Bereich 11 mit vermindertem Leitungsquerschnitt mit den Elektroden 8 verbunden sind, wie aus Fig. 6 deutlicher erkennbar ist.

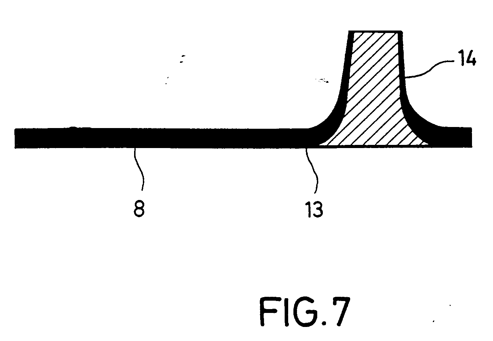

Ein Wärmetauscher 12 besteht aus zwei aufeinandergelegten ringförmigen Abschnitten 13, die einen tiefgezogenen Bereich 14 aus dem gleichen metallischen Material aufweisen, aus dem auch die Elektrode gebildet ist. Wie aus Fig. 7 deutlich wird, beträgt das Verhältnis der Länge des tiefgezogenen Bereichs 13 zur Dicke der Elektroden in etwa 6:1. Der Tiefziehvorgang wird so durchgeführt, daß sich die Materialstärke des tiefgezogenen Bereichs zu seinem freien Ende hin verjüngt. Insbesondere ist es bei Tiefziehen wichtig, daß ausreichend Material an der Ansatzstelle zwischen dem tiefgezogenen Bereich 14 und dem ringförmigen Abschnitt 13 vorhanden ist, damit ein guter Wärmeübergang auf dem tiefgezogenen Bereich gewährleistet ist. Die größere Materialstärke an den Stellen, die nahe am Zeizelement liegen, führt dazu, daß die Heizenergie gut bis an die äußeren Enden übertragen wird. Der ringförmige und tiefgezogene Bereich 13, bzw. 14 sind vollständig in dem isolierten Kunststoffmaterial eingebettet. In Fig. 3 erkennt man die umspritzten, ringförmigen und tiefgezogenen Bereiche des Wärmetauschers 12, der einen axial zentrierten Durchgangskanal 12a aufweist. Der ringförmige Bereich ist an seiner Innenwand nur mit einer dünnen Isolierschicht überzogen, damit möglichst viel Wärmeenergie in den Durchgangskanal 12a abgeführt werden kann. Dagegen besitzt die Außenwand des ringförmigen Bereichs eine dicke Isolierschicht, die sich in axialer Richtung des tiefgezogenen Bereichs zu seinem freien Ende hin verstärkt. Eine so ausgebildete Isolation vermindert die Wärmeverluste und sorgt dafür, daß die zu übertragende Wärme genau an die gewünschte Stelle im Durchgangskanal transportiert wird.

Im folgenden sollen die wesentlichen Schritte zur Herstellung einer Vorrichtung nach der vorliegenden Erfindung dargestellt werden.

Fig. 6 zeigt ein Stanzblechteil 15 mit den wesentlichen Teilen der elektrischen Heizung. Im vollständig ausgestanzten Zustand des Blechteils sind die Stellen, die in der Zeichnung mit gestrichelten Linien gekennzeichnet sind, vollständig durchtrennt. Im einzelnen erkennt man zwei Elektroden 8 mit daran integral ausgeformten ringförmigen Abschnitten 13, die jeweils einen tiefgezogenen Bereich 14 aufweisen. Integral mit den Elektroden 8 bzw. dem ringförmigen Abschnitt 13 sind Verbindungsleiter 10 verbunden, zum Teil über Bereiche 11 mit vermindertem Querschnitt. Die Verbindungsleiter sind in der linken Hälfte des in Fig. 6 gezeigten Stanzteils etwa viertelkreisförmig und einander zugewandt ausgebildet und tragen jeweils ein Paar jeweils um 90° versetzte Kontakte 16.

In einem weiteren Verfahrensschritt wird das Stanzblechteil an den Stellen, an denen eine erhöhte Formsteifigkeit bzw. eine Wärmeisolierung erforderlich ist, mit einem Kunststoff umspritzt. Die Umspritzung der Viertelkreisringe wird so vorgenommen, daß sich ein nahezu geschlossener Kreisring ausbildet, an dessen Innenwand die Kontakte 16 radial nach innen ragen. An der Stirnseite des Kreisrings ist ein Steg 17 ausgebildet, der sich in axialer Richtung des Kreisrings erstreckt. Der Steg verläuft auf einer Länge von ca. einem Viertel des Kreisrings und weist in seiner Innenwandung eine Vielzahl von Vertiefungen 18 mit abgeschrägten Flächen auf (Fig. 3). Nach dem Umspritzen werden die in Fig. 6 mit gestrichelten Linien gezeichneten Stellen durchtrennt, wodurch die in der linken Hälfte gezeigte Elektrode vollständig herausfällt. Danach werden die in dem nahezu geschlossenen Kreisring eingebetteten viertelkreisringförmigen Verbindungsleiter an den Stellen 19 um etwa 90° gegenüber dem verbleibenden elektrischen Teil der Heizung abgebogen, was aus Fig. 2 ersichtlich ist.

Schließlich wird die Heizung mit Widerständen 20 und einer Leuchtdiode 21 vollständig bestückt.

Im Betrieb der Vorrichtung funktioniert der elektrische Schaltkreis der Heizung wie folgt: Ausgehend von einer Verbindungsleitung 10 fließt der Heizstrom über einen Sicherungswiderstand 20 zum oberen kreisringförmigen Abschnitt 13, der integral mit der oberen Elektrode 8 ausgebildet ist. Von dieser Elektrode fließt der Strom durch das PTC-Heizelement hindurch zur unteren Elektrode 8 und von dort zu einer zweiten Verbindungsleitung 10, welche vollständig vom Kunststoffmaterial umspritzt ist. Die beiden Verbindungsleitungen 10 sind mit den in Fig. 1 gezeigten Steckkontakten 6 des Anschlußsteckers 5 verbunden. Parallel zu dem Elektrodenpaar 8 ist ein Vorwiderstand 20 mit einer dazu in Reihe geschalteten Leuchtelektrode 21 geschaltet. Die Verbindungsleitungen für den Vorwiderstand und die Leuchtelektrode befinden sich jeweils an dem oberen und unteren kreisringförmigen Abschnitt 13 des Wärmetauschers 12.

PTC-Heizelemente sind dafür bekannt, daß sie sich bei einer von ihrer Dimensionierung abhängigen Temperatur selbst stabilisieren. Für das vorliegende Ausführungsbeispiel wurde das PTC-Heizelement so ausgewählt, daß es bei der vorgegebenen Betriebsspannung eine Temperatur von 150°C erzeugt. Da sich die obere und untere Elektrode 8 in innigem Kontakt mit dem Heizelement befindet und die ringförmigen Abschnitte mit dem tiefgezogenen Bereich einstückig daran ausgebildet sind, wird die vom Heizelement erzeugte Wärme zum Wärmetauscher 12 transportiert. Aufgrund der erfindungsgemäßen Ausgestaltung der Vorrichtung sind die Wärmeverluste so gering, daß in dem Durchgangskanal am Docht eine Temperatur von ca. 128° gemessen wurde. Bemerkenswert an diesem Ergebnis ist der geringe Temperaturverlust von nur 22°C gegenüber der Betriebstemperatur des PTC. Weiterhin ist bei Dauerversuchen eine sehr geringe Temperaturschwankung von nur 0,5°C nachgewiesen worden.

Es soll darauf hingewiesen werden, daß die dargestellte Ausführung der Heizung nur eine beispielhafte Möglichkeit darstellt und das vielmehr mehrere technische Realisierungsmöglichkeiten für die Heizung existieren. Zum Beispiel muß das Heizelement nicht unbedingt aus einem PTC-Heizelement bestehen, sondern kann auch eine Heizwicklung aus einem Heizdraht sein, die in einem keramischen Heizkörper eingebettet ist.

Eine andere, in den Zeichnungen nicht dargestellte Ausführungsform der vorliegenden Erfindung, unterscheidet sich von der oben beschriebenen Ausführung durch eine andere Ausbildung des Wärmetauschers 8. Bei dieser Ausführungsform werden die kreisringförmigen Abschnitte 13 und der tiefgezogene Bereich 14 durch eine ebene Heizfläche ersetzt. Weiterhin wird anstelle des Behälters 2 mit dem darin gehaltenen Docht ein Behälter vorgesehen, der sich oberhalb der Heizfläche befindet, beispielsweise eine wärmebeständige Plastikschale. In der Schale befindet sich die zu verdunstende Substanz, welche direkt von der darunter angeordneten Heizfläche gleichmäßig beheizt wird. Bei diesem Ausführungsbeispiel weist das Gehäuse eine geeignete Öffnung auf, durch die der Behälter mit der zu verdunstenden Substanz eingebracht werden kann.

Im folgenden soll Bezug genommen werden auf die Fig. 4 und 5, welche die Ausbildung eines ersten Drehkupplungsteils 22 erläutern.

Das erste Drehkupplungsteil 22 ist starr mit dem Anschlußstecker 5 verbunden und besteht im wesentlichen aus einem scheibenförmigen Trägerteil 23, welches zwei sich gegenüberstehende streifenförmige elektrische Kontakte 24 trägt. Die elektrischen Kontakte 24 sind über Kontaktstifte 25 (Fig. 5) mit den Steckkontakten 6 verbunden. Am äußeren Umfang des scheibenförmigen Trägerteils 23 ist ein Nocken 26 ausgebildet, der an seinem freien Ende zwei Flächen aufweist, die einen Winkel von etwa 120° miteinander einschließen.

Ein zweites Drehkupplungsteil 27 besteht im wesentlichen aus dem nahezu geschlossenen Kreisring, der aus der Umspritzung der viertelkreisringförmig ausgebildeten Verbindungsleitungen 10 entstanden ist (vgl. Fig. 2 und 3) und aus dem damit verbundenen Steg 17, der eine Anzahl Vertiefungen aufweist.

Wenn die erfindungsgemäße Vorrichtung fertig montiert ist, greift das Trägerteil 23 des ersten Drehkupplungsteils 22 in das kreisringförmige zweite Drehkupplungsteil 27 ein. Der an dem Trägerteil 23 ausgebildete Nocken 26 greift dabei in die Vertiefungen 18 ein, die an dern Innenwandung des zweiten Drehkupplungsteils ausgebildet sind. Wenn der Anschlußstecker 5 mit dem daran befestigten ersten Drehkupplungsteil gegenüber dem Gehäuse gedreht wird, gleitet der Nocken 26 rastend über die Vertiefungen 18 des zweiten Drehkupplungsteils. Die Verdrehung des Anschlußsteckers 5 gegenüber dem Gehäuse ist mittels einer nicht näher gezeigten Begrenzungseinrichtung auf etwa 90° begrenzt.

## Patentansprüche

1. Elektrische Vorrichtung zum Verdunsten von Wirkstoffen, Parfümen od. dgl. flüchtige Substanzen, mit einem Gehäuse und einer darin gelagerten elektrischen Heizung, die ein Heizelement und einen Wärmetauscher umfaßt, um die von dem Heizelement erzeugte Wärme dem Wärmetauscher zuzuführen und dort an die zu verdunstende Substanz abzugeben, **dadurch gekennzeichnet,** daß
das Heizelement aus einem PTC-Element besteht, welches zwischen zwei Elektroden (8) eingeklemmt ist, und daß wenigstens eine der beiden Elektroden (8) nahe dem Wärmetauscher (12) angeordnet und einstückig mit diesem ausgebildet ist.

2. Vorrichtung nach Anspruch 1, **dadurch gekenzeichnet,** daß ein mit dem Gehäuse (1a, 1b) verbindbaren Behälter (2) für die zu verdunstende Substanz vorgesehen ist, daß in dem Behälter ein Docht (3) gelagert ist, und daß der Wärmetauscher (12) einen Durchgangskanal (12a) aufweist, der den Docht (3) umschließt.

3. Vorrichtung nach Anspruch 2, **dadurch gekennzeichnet,** daß der Wärmetauscher (12) ringförmig ausgebildet ist mit einem tiefgezogenen Abschnitt (14), der sich koaxial zum Docht (3) erstreckt.

4. Vorrichtung nach Anspruch 1, **dadurch gekennzeichnet,** daß der Wärmetauscher (12) als eine ebene Heizfläche ausgebildet ist, die als Auflagefläche für einen Behälter für die zu verdunstende Substanz dient.

5. Vorrichtung nach einem der Ansprüche 1 bis 4, **dadurch gekennzeichnet,** daß die Elektroden (8) und/oder der Wärmetauscher (12) mit einstückig daran ausgebildeten Verbindungsleitungen (10) versehen sind, um eine elektrische Verbindung zwischen der Heizung (4) und einem Anschlußstecker (5) herzustellen.

6. Vorrichtung nach Anspruch 5, **dadurch gekennzeichnet,** daß die Verbindungsleitungen (10), der Wärmetauscher (12) und die Elektroden (8) einstückig auf einem Stanzblechteil (15) gebildet sind, das nach dem Stanzen erforderlichenfalls abgebogen und an den Stellen, an denen eine erhöhte Formsteifigkeit bzw. eine Wärmeisolierung erforderlich ist, mit einem Kunststoff umspritzt ist.

7. Vorrichtung nach Anspruch 5 oder 6, **dadurch gekennzeichnet,** daß ein Bereich (11) mit vermindertem Leitungsquerschnitt zwischen den Verbindungsleitungen (10) und den Elektroden (8) oder dem Wärmetauscher (12) ausgebildet ist.

8. Vorrichtung nach Anspruch 6, **dadurch gekennzeichnet,** daß die Elektroden (8) auf den voneinander abgewandten Seiten von dem Kunststoff umgeben sind und auf den, dem PTC-Element zugewandten Seiten einen Steg aufweisen, der am äußeren Rand der Elektrode (8) ausgebildet ist und daß das PTC-Element zwischen den Stegen formschlüssig gehalten ist.

9. Vorrichtung nach wenigstens einem der Ansprüche 1 bis 8, **dadurch gekennzeichnet,** daß ein elastisches Element zwischen einer Elektrode (8) und dem PTC-Element angeordnet ist und daß ein Klemmelement (9) von außen die zwei Elektroden (8) umgreift und das PTC-Element in engem Kontakt zu den Elektroden (8) hält.

## Claims

1. Electric device for the vaporization of active substances, perfumes or similar volatile substances, comprising a housing and an electric heating disposed therein, said heating comprising a heating element and a heat exchanger, to supply the heat generated by the heating element to the heat exchanger, and to convey it there to the substance to be vaporized, **characterized in** that
the heating element consists of a PTC element, clamped between two electrodes (8), and that at least one of the two electrodes (8) is disposed in proximity to the heat exchanger (12) and is integrally formed therewith.

2. A device according to claim 1, **characterized in** that a container (2), connectable to the housing (1a, 1b) is provided for the substance to be vaporized, that a wick (3) is held within the container, and that the heat exchanger (12) comprises a passage channel (12a), which surrounds the wick (3).

3. A device according to claim 2, **characterized in** that the heat exchanger (12) is formed annularly having a deep drawn section (14), extending coaxially to the wick (3).

4. A device according to claim 1, **characterized in** that the heat exchanger (12) is provided as a planar heating surface, which serves as a support surface for a container for the substance to be vaporized.

5. A device according to one of claims 1 to 4, **characterized in** that the electrodes (8) and/or the heat exchanger (12) are provided with connecting strips (10) formed integrally therewith, to establish an electrical connection between the heating (4) and a plug (5).

6. A device according to claim 5, **characterized in** that the connecting strips (10), the heat exchanger (12) and the electrodes (8) are integrally formed on a punching sheet member (15), which is bent after punching, if necessary and which is coated by a plastic material at the points at which an increased inherent stability or heat insulation is required.

7. A device according to claim 5 or 6, **characterized in** that a region (11) having a reduced conductive cross section is provided between the connecting strips (10) and the electrodes (8) or the heat exchanger (12).

8. A device according to claim 6, **characterized in** that the electrodes (8) are surrounded by the plastic material on the sides facing away from one another, and comprise a web on the sides facing the PTC element, formed at the outer edge of the electrode (8) and that the PTC element is held in form-fit fashion between the webs.

9. A device according to one of claims 1 to 8, **characterized in** that a resilient element is disposed between an electrode (8) and the PTC element, and that a clamping element (9) surrounds the two electrodes (8) from the outside and keeps the PTC element in close contact to the electrodes (8).

## Revendications

1. Appareil électrique pour diffuser des substances actives, des parfums ou des substances volatiles similaires, comportant un boîtier dans lequel se trouve un dispositif de chauffage électrique, qui comporte un élément chauffant et un échangeur de chaleur, pour conduire la chaleur dégagée par l'élément chauffant vers l'échangeur de chaleur et la transmettre ensuite à la substance à diffuser, caractérisé en ce que l'élément chauffant est constitué par un élément à coefficient de température positif (CTP) qui est inséré entre deux électrodes (8), et en ce que l'une au moins des deux électrodes (8) est agencée à proximité de l'échangeur de chaleur (12), dont elle est solidaire.

2. Appareil selon la revendication 1, caractérisé en ce qu'un conteneur (2) associé au boîtier (1a, 1b) est prévu pour la substance à diffuser, en ce qu'une mèche (3) est placée dans le conteneur, et en ce que l'échangeur thermique (12) comporte un passage (12a) dans lequel est enfermée la mèche.

3. Appareil selon la revendication 2, caractérisé en ce que l'échangeur thermique (12) a une forme circulaire et présente une section emboutie (14) qui s'étend coaxialement à la mèche (3).

4. Appareil selon la revendication 1, caractérisé en ce que l'échangeur thermique (12) se présente sous la forme d'une surface chauffante plane qui sert de surface d'appui pour un conteneur destiné à la substance à diffuser.

5. Appareil selon l'une quelconque des revendications 1 à 4, caractérisé en ce que les électrodes (8) et/ou l'échangeur de chaleur (12) sont pourvus de circuits de liaison (10) qui en sont solidaires, pour réaliser un raccordement électrique entre le dispositif de chauffage (4) et une fiche de raccordement (5).

6. Appareil selon la revendication 5, caractérisé en ce que les circuits de liaison (10), l'échangeur de chaleur (12) et les électrodes (8) sont formées solidairement sur une pièce d'emboutissage (15) qui est pliée en cas de besoin après l'emboutissage et qui est recouverte par extrusion d'une matière plastique aux endroits où une rigidité de forme, ou une isolation thermique, majorée est nécessaire.

7. Appareil selon la revendication 5 ou 6, caractérisé en ce qu'une zone (11) de section réduite est formée entre les circuits de liaison (10) et les électrodes (8) ou l'échangeur de chaleur (12).

8. Appareil selon la revendication 6, caractérisé en ce que les électrodes (8) sur les côtés opposés sont entourées de matière plastique et comportent sur les côtés orientés vers l'élément CTP une entretoise qui est réalisée sur le bord extérieur de l'électrode (8) et en ce que l'élément CTP est maintenu en engagement positif entre les entretoises.

9. Appareil selon l'une quelconque des revendications 1 à 8, caractérisé en ce qu'un élément élastique est agencé entre une électrode (8) et l'élément CTP et en ce qu'un élément de serrage (9) enserre de l'extérieur les deux électrodes (8) et maintient l'élément CTP en contact étroit avec les électrodes (8).
